# EUROPEAN PATENT APPLICATION

(11) **EP 4 674 261 A1**
(43) Date of publication of application: **07.01.2026**
(21) Application number: 24185846.3
(22) Date of filing: 01.07.2024
(51) Int. Cl.: A01K 67/033

(54) **A DOSING DEVICE FOR INSECTS**

(71) Applicant: Enorm Biofactory A/S, 8762 Flemming (DK)
(72) Inventor: Winther, Marius Løssl, 8762 Flemming (DK); Schou, Toke Munk, 8762 Flemming (DK); Hougaard, Per, 8762 Flemming (DK)
(74) Representative: Dragsted Partners A/S

(57) **Abstract**

A dosing device for insects, the insects having a first size, the dosing device comprising a dosing body having a central axis, having a first end facing the dosing device and a second end, a dosing volume having a longitudinal axis, defined at least partly by the dosing body, having a dosing opening arranged at the second end of the dosing body, a first valve device configured to control a passage of air into and/or out of the first end of the dosing body and/or into and/or out of the dosing volume, where the first valve device is configured to be in fluid communication with a first air source, and a filter part having a first surface facing the dosing opening and a second surface facing away from the dosing body, the first surface defining a boundary of the dosing volume, and the first end part having at least one through-going opening configured to allow air to, while preventing the passage of insects.

## Description

### Technical field

A dosing device for insects, the insects having a first size, and the dosing device comprising a dosing body having a central axis having a first end facing the dosing device and a second end, a dosing volume having a longitudinal axis, defined at least partly by the dosing body having a dosing opening arranged at the second end of the dosing body.

### Description

There are a number of different challenges that can affect the current climate, where it is widely recognized that there is too much CO2 in the atmosphere, and that traditional agricultural methods and protein production may contribute to a large part of the release of CO2 and other greenhouse gasses into the atmosphere. Thus, there is a need to identify agricultural methods that may be developed to reduce the impact on our atmosphere and environment.

Considering climate change and the increasing world population, effective circularity and sustainable food production have never been more crucial. To alleviate the strain that food production imposes on natural resources, the production of insects for food production can play a pivotal role, where the production of Black Soldier Fly larvae may reduce the need for traditional food production or improve/optimize agricultural methods and provide an environmentally friendly production of ingredients for both feed and food.

The production of Black Soldier Fly larvae is a process in which the yield of useable nutrients from the larvae is very high in comparison to the resources needed to produce the larvae. However, in order to have an effective process for producing larvae, there is a need for high precision in the dosing of the larvae and the feed necessary to grow the larvae. Thus, in order to have an effective growing process, it is very important to provide a precise amount of feed for a precise amount of larvae.

In the process, it is relatively easy to dose the feed for the larvae, as the feed may be weighed precisely using traditional scales. However, one of the more difficult issues is to dose the neonates that grow into larvae. When dosing neonates, it is important that the dosage matches the dosage of feed. The dosing of neonates is often done by way of a specific number of neonates that matches the amount of feed. The precise number of neonates may be adjusted for the specific amount of neonate feed. If there are too many neonates in relation to the amount of food, the growth of the neonates is suppressed, and the size of the resulting larvae batch is not uniform, as some larvae have received more feed than others. If the amount of feed is too large compared to the number of larvae, the feed has not been consumed when the larvae are to be harvested, which prevents the harvesting of the larvae or makes it difficult and not effective. Thus, non-precise dosing methods may result in significant deviations in the yield between crates and between batches as well as deviations in larvae size, all of which results in a negative effect on the feed conversion ratio of the larvae, and the yield between crates and between batches may differ significantly.

Thus, it is important to match the number of neonates with the amount of feed to optimize the production and the growth rate of the larvae.

The counting of neonates, which are extremely small, sticky and tend to cluster, can be challenging, and incorrect counting or insufficient weight dosing may lead to considerable errors. Additionally, prior art solutions that attempt to increase dosing accuracy are typically very slow and consequently not cost-effective. This is true not only for neonates, but also for other insects with similar characteristics, i.e. very small insects with large variations in size for different samples and a tendency to aggregate.

Thus, there is a need to improve the dosing of neonates in order to improve the efficiency and efficacy of larvae production.

In accordance with the invention, there is provided a dosing device for insects, the insects having a first size, and the dosing device comprising a dosing body having a central axis having a first end facing the dosing device and a second end, a dosing volume having a longitudinal axis, defined at least partly by the dosing body having a dosing opening arranged at the second end of the dosing body, a first valve device configured to control a passage of air into and/or out of the first end of the dosing body and/or into and/or out of the dosing volume, where the first valve device is configured to be in fluid communication with a first air source, and a filter part having a first surface facing the dosing opening and a second surface facing away from the dosing body, the first surface defining a boundary of the dosing volume, and the first end part having at least one through-going opening configured to allow air to pass, while preventing the passage of insects.

In larvae production, there are numerous stages in the growth of the larvae from the harvesting of insect eggs, where each step takes a certain amount of time, and where the length of each stage may be manipulated by controlling the temperature in the production phase.

The insect eggs may be harvested from insect cages, where the insects have laid their eggs in predefined egg collectors that may be removed from the fly cage and replaced by new egg collectors. The eggs may be placed in a storage space where the embryonic development may be controlled by setting the correct temperature in the egg storage. The eggs may subsequently be positioned in a second storage in which the temperature may be set to induce the hatching of the eggs by optimizing the embryonic development and the maturation of the eggs.

When the eggs are hatched, the larvae are in their neonatal stage, the neonatal larvae being called neonates. The neonates may be introduced into a third storage that may be utilized to store the neonates at a predefined temperature in order to maintain the size of the neonates after hatching. The third storage may be utilized to gather neonates and to attempt to maintain the size of the neonates before they are transferred to their growth feed. In this storage room, the growth of the neonates may be controlled by temperature and/or humidity in order to ensure that all neonates have a similar size, or at least have a size dispersion that is predictable and repeatable by repeating the hatching process and the storage of the neonates. This ensures even survival, size and texture between batches of neonates.

When the neonates are to be utilized for growth into larvae that are usable for agriculture and/or food production, the neonates may be transported into a growth environment where the neonates are introduced into containers with feed to allow the neonates to grow. When this occurs, it is important that the number of neonates introduced into the containers with growth feed is predictable, and that the amount of feed is adjusted to the number of neonates.

The use of the dosing device in accordance with the present disclosure makes it possible to provide a very precise dosage for neonates, where the neonates are introduced into the dosing volume of the dosing device, and where the size of the volume of the dosing device defines how many neonates are arranged within the dosing volume.

By utilizing the valve device, it is possible to utilize air to introduce the neonates into the dosing volume, where neonates are introduced into the dosing volume via the dosing opening, which is arranged close to or at the second end of the dosing body, and where the side walls of the dosing body and the filter device define the size of the dosing volume.

The first valve device may be utilized to control the passage of air into the first end of the dosing body and thereby also the dosing volume, where the flow of air into the dosing body sucks the neonates into the dosing volume. The predefined size of the dosing volume ensures that the flow of air sucks a predefined number of neonates into the dosing volume until the dosing volume is full. The size of the neonates and the size of the dosing volume may be matched so that when the dosing volume is full, a predefined number of neonates are inside the dosing volume. Thus, by controlling the size of the dosing volume and using air flow to introduce the insect neonates into the volume, it is possible to determine the number of neonates inside the dosing volume and/or the dosing body.

The use of air to introduce the neonates into the dosing volume means that the neonates are introduced using a flow of air into the dosing volume, where the pressure of the air may affect the level of packing of the neonates inside the dosing volume. If a high volume with high pressure is used to introduce the neonates into the dosing volume, the neonates may be packed at a predictable and repeatable rate, so that a predefined volume has a predefined number of neonates. If the pressure and/or the flow of air is changed, the packing of the neonates may be altered inside the dosing volume, and the number of neonates may be altered.

The valve device may be any kind of valve device which allows air passage in and out of the dosing volume. The valve device may be positioned together with the dosing body and the filter device, or may be connected via hoses to the valve device and/or the dosing body. The valve device may be in the form of one valve that may be upstream from the dosing body, allowing the valve device to feed air into the dosing body, or may be downstream to the dosing body, allowing air to be sucked into the dosing body before entering the valve device. The valve device may be in the form of two valves, where one valve provides an air flow into the dosing body from the first end to the second end, while the other valve provides a vacuum into the dosing body, allowing air to flow into the dosing body from the second end to the first end.

The packing of neonates, and therefore the number of neonates, may be a function of the dosing volume but may also be affected by the time intervals of air passage, or bt the time during which the dosing device is arranged in a batch of neonates under vacuum. The number may be dependent on whether the air (vacuum) is turned on before the dosing part enters the batch of neonates when entering the batch of neonates or whether the flow of air is delayed until the dosing part is fully submerged in the batch of neonates.

The dosage device may be utilized for any-sized neonates, where the size of the dosing volume may be adjusted to the size of the neonates that are to be introduced into the dosing volume.

The filter part may be utilized to define one boundary of the dosing volume, where the flow of air may pass through the filter part, thereby allowing the air to push the neonates against the first surface, and where the first surface allows the air to pass through, while preventing the neonates from passing through the filter part in a direction from the first surface to the second surface. Thus, the air flow through the filter device allows the neonates to be packed inside the dosing volume. The air flow should be controlled so that the neonates inside the dosing volume do not prevent the flow of air into the dosing volume.

When the dosing volume is full, the dosing device may be utilized to reverse the air flow, so that the air flow direction is from the first end towards the second end of the dosing body, and in a direction towards the dosing opening. Thus, the air flow may be utilized to push the neonates out of the dosing volume, where the air flow pushes all of the neonates out of the dosing volume. Thus, the flow of air may be through the filter device in a direction from the second surface to the first surface of the filter device, where the flow of air may clear the neonates out of the dosing volume from the first surface of the dosing device. The air may flow through the at least one through-going opening, pushing the neonates out of the dosing opening.

Thus, after the hatching process, a plurality of neonates may be collected at a first position, such as a neonate container, or on a surface having a plurality of neonates, where the dosing device may be used to collect the neonates into the dosing volume, allowing a predefined number of neonates to be collected from the neonate container, and where the neonates are moved from inside the dosing volume to a second position, where the predefined number of neonates collected inside the dosing volume may be dispensed at the second position, such as into a vial or a vessel. The vial or vessel has a predefined number of neonates, which makes it easier to transport the neonates to the position where the neonates are intended to grow into larvae, and makes it easier to dose the feed in accordance with the number of neonates in the vial and/or vessel.

The vial/and or vessel may comprise a substrate that may be optimized to keep the neonates alive during transport without initiating the growth of the neonates into full-size larvae. The growth rate of the neonates may be suspended by adjusting the temperature of the neonates in a fourth storage. Thus, the neonates may be suspended for days or weeks until they are ready to go into their larvae growth stage.

Alternatively, the dosing device may be utilized to dose neonates directly to the growth feed or to a biological substrate, where the neonates are not positioned in a vessel, such as a cup, container, box, etc.

Thus, by utilizing a dosing device in accordance with the present disclosure, the neonates may be dosed at a predictable accuracy, and fast, as the introduction of neonates into the dosing volume may be done within seconds, and the expulsion/ejection of the neonates from the dosing volume may be done within seconds. Specifically, the introduction and the expulsion of the neonates may be done within one second, respectively, or more specifically within a range of 0.2-0.8 seconds, respectively. In prior art solutions, the counting or weighing of neonates is a slow and time-consuming process, where the stickiness of the neonates complicates the counting or weighing process, and where the movement of the neonates during the counting and weighing may reduce the accuracy of the weight or the accuracy of the counting.

By utilizing air to pack neonates into a dosing volume, the air may reduce the movement of the neonates and the packing with air may benefit from the neonates sticking to each other, as it is advantageous to have a predefined packing rate inside the dosing volume.

In one or more exemplary embodiments, the insects may be in the form of neonates. This means that the dosing may be performed on insects in a neonatal stage of their growth, which neonatal stage is subsequent to the hatching stage of an insect egg.

In one or more exemplary embodiments, the at least one through-going opening has an opening diameter that is smaller than the first size of the insect. By providing a through-going opening that has an opening diameter that is smaller than the first size of the insect, the insect cannot pass through the through-going opening, and the filter part will prevent the insect from being pulled out of the dosing volume. This may be advantageous in two ways, in that, first, the neonates that are moved inside the dosing volume via the dosing opening cannot exit the dosing volume via any other means, thereby ensuring that all insects that are drawn into the dosing volume stay inside the dosing volume while the air is actively being drawn into the dosing volume via the dosing opening. Second, the size of the opening ensures that the insects that are drawn into the dosing volume are not pulled from the dosing volume and into the valve device and/or any air hoses, ensuring that the insects do not damage or block the flow of air via the valve device.

In one or more exemplary embodiments, the dosing body may be a cylindrical body, where the body has an outer surface defining an outer periphery of the dosing body, and where the dosing body may have a cylindrical inner surface which may define an outer radial boundary of the dosing volume. Thus, an inner surface of the dosing body may define the size of the dosing volume, and the dosing body may have a second end that defines the dosing opening. Thus, the dosing volume may have a cylindrical shape, where the first end of the dosing volume is defined by the filter part, and the radial boundary of the dosing volume is defined by the inner surface of the dosing body and may be cylindrical in shape along the longitudinal length of the dosing body. Access may be made into the dosing volume via the dosing opening, which may be defined by the second end of the dosing body.

In one or more exemplary embodiments, the filter part is arranged in fluid communication between the first valve device and/or the first dosing volume and/or the dosing body. This means that the filter part may be arranged in a position that may be downstream to the first dosing volume and/or the dosing body when the air passes the dosing volume and/or the dosing body on its way to the valve device, or may be upstream to the dosing volume and/or dosing body when the passage of air moves from the valve device and towards the dosing volume and/or the dosing body, thereby allowing the insects to be discharged out of the dosing volume. Thus, the filter part may be arranged in the fluid communication pathway between the first valve device and the dosing volume and/or the dosing body.

In one or more exemplary embodiments, the filter part is removably attached to the dosing part and or the dosing device. By having the filter part removably attached to the dosing part and/or the dosing device, it may be possible to disassemble the filter part from the dosing device and/or the dosing device, where the filter device may be cleaned and reassembled with the dosing part and/or the dosing device, as the insects may clog up one or more of the through-going openings of the filter part, or where lipids secreted by the neonates clog up the one or more through-going openings.

Alternatively, should the dosing device be used for a different insect, or a different size insect be intended to be dosed, the filter part may be replaced by a filter part having different properties, such as different diameter of through-going openings, or alternatively the filter part may be replaced by an identical filter part that is clean and ready to be utilized for the dosing device.

In one or more exemplary embodiments, the filter part is in the form of a mesh-like structure having a plurality of through-going openings of substantially the same size. The filter part may have a plurality of through-going openings that all have a similar size, or substantially the same size, where each of the through-going openings is configured to allow the air to pass between the valve device and the dosing volume and/or the dosing part. Thus, even if one through-going opening is blocked, the air may pass through other through-going openings.

In one or more exemplary embodiments, the first valve device has an air supply inlet and a dosing device outlet and/or a dosing volume outlet. The first valve device may be configured to be connected to an air supply via the air supply inlet, where the first valve device is configured to selectively open up for a flow of air, allowing air to pass from the first valve device into the dosing device outlet and/or the dosing volume outlet, where the air passing the dosing device outlet and/or the dosing volume outlet is directed into or out of the dosing volume and/or the dosing body.

In one or more exemplary embodiments, the first valve device has a vacuum source inlet, where the first valve device may selectively open up fluid communication between the vacuum source inlet and the dosing device outlet and/or the dosing volume outlet. This allows the valve to open up for a vacuum in order to allow the air to be sucked into the dosing volume via the dosing opening.

In one or more exemplary embodiments, the first valve device may be a valve device that is configured to be connected to an air source, where the first valve device may be configured to modify the first air source to a vacuum source, and where the first valve device is capable of provide an air source. Thus, the valve device may be capable of converting positive air pressure to negative air pressure, where the negative air pressure of the valve device is in fluid communication with the dosing device outlet and/or the dosing volume outlet, and the valve device may also be capable of transferring positive pressure which is in fluid communication with the dosing device outlet and/or the dosing volume outlet. The valve device may therefore be capable of providing the dosing device outlet and/or the dosing volume outlet with both selective positive air pressure and negative air pressure.

In one or more exemplary embodiments, the first valve device in a first operational state is configured to provide air pressure that is lower than the ambient air pressure. The first valve device may in a first operational state provide the dosing volume with air pressure that is lower than the ambient air pressure, allowing the air pressure to provide a suction force into the dosing volume, and thereby allowing the insects to be collected into the dosing volume.

In one or more exemplary embodiments, the first valve device in a second operational state is configured to provide air pressure that is higher than the ambient air pressure. The first valve device may in a first operational state provide the dosing volume with air pressure that is higher than the ambient air pressure, allowing the air pressure to provide a blowing force into the dosing volume, thereby allowing the insects to be blown or pushed out of the dosing volume.

In one or more exemplary embodiments, the first valve device, in a first operational state, is configured to produce a vacuum which initiates a flow of air from the second end of the dosing body and/or the dosing opening of the dosing volume in a direction towards the filter part and/or the valve device. This means that the flow of air is in a direction that is from the second end of the dosing body to the first end of the dosing body, allowing the flow of air to flow into the dosing opening.

In one or more exemplary embodiments, the first valve device, in a second operational state, is configured to produce a flow of air from the filter part and/or the valve device in a direction towards the second end of the dosing body and/or the dosing opening of the dosing volume. This means that the flow of air is in a direction from the first end of the dosing body and/or the dosing volume to the second end of the dosing body and/or the dosing volume, allowing the air to flow out of the dosing opening of the dosing volume.

In one or more exemplary embodiments, the dosing volume has a longitudinal length that is larger than the diameter of the dosing volume. The dosing volume may be arranged in such a way that the length of the dosing volume along the longitudinal axis of the dosing volume is larger than the radial width of the dosing volume. The size of the dosing volume may be defined by the length of the dosing volume and the diameter of the dosing volume. This configuration is shown as having a high rate of repeatability of filling the dosing volume with a predefined number of insects, as the air flows along the length of the dosing volume, which is larger than the width of the dosing volume. This ensures that the flow of air is relatively uniform along the entire radial diameter of the dosing volume, and that the insects will pack in a predetermined manner inside the dosing volume.

In one or more exemplary embodiments, the dosing volume and/or the dosing body has a cylindrical shape. At least part of the dosing volume may have a cylindrical shape. This means that the cylindrical shape may have a predefined number of insects in a predefined length of the dosing volume. Furthermore, the cylindrical shape ensures that the inner surface of the dosing body, which defines the radial boundary of the dosing volume, may easily be emptied of insects when the insects are pushed out of the dosing volume using an air flow.

In one or more exemplary embodiments, the dosing volume and/or the dosing body has a first area having a first diameter and a second area having a second diameter, where the first diameter is different from the second diameter. By having two different diameters in a first area vs. a second area, it may be possible to distribute the air flow in a different manner in different parts of the dosing volume. The first diameter may be arranged in a longitudinal position that is different from the longitudinal position of the second diameter. Thus, the suction or the blowing of air to and from the dosing volume may be controlled differently in different areas of the dosing volume.

Furthermore, in one exemplary embodiment the dosing opening may have a radial diameter that is different from the radial diameter of the dosing volume, where the size of the opening may be smaller than the size of the dosing volume in order to allow the air to flow faster through the dosing opening while flowing slower inside the dosing volume in order to allow the slower air to pack the insects in predefined manner without damaging the insects due to the slower air flow inside the dosing volume. When the dosing volume is full, i.e. when the number of insects inside the dosing volume has reached its predefined number, the air may maintain a plurality of insects in the vicinity of the opening, but outside the dosing volume. This has been shown as being a number of insects that are being affected by the suction of air, and where the insects form a bulge outside the dosing opening. By reducing the size of the dosing opening, this bulging may be reduced, thereby increasing the consistency of the number of insects being picked up by the dosing device.

The present disclosure also relates to a dosing system comprising a dosing device in accordance with the above and a first air source in fluid communication with the first valve device. The dosing system may include an air source such as an air pump and/or a vacuum pump, where the air source may be in fluid communication with the valve device to provide air flow through the dosing volume and/or the dosing body in a direction into the dosing volume via the dosing opening, and in a direction out of the dosing volume via the dosing opening.

### Brief description of the drawings

The following is an explanation of exemplary embodiments with reference to the drawings in which:
Fig. 1 is a cross-sectional view of a dosing device in accordance with the present disclosure,
Fig. 2 is a side view of a filter part in accordance with the present disclosure,
Fig. 3 is a top view of a filter part in accordance with the present disclosure,
Fig. 4 is a side view of a filter part and a dosing body in accordance with the present disclosure,
Fig. 5 is a side view of a dosing device in accordance with the present disclosure,
Fig. 6 is a front view of a dosing device in accordance with the present disclosure, and
Fig. 7 is a front view of a dosing device attached to a robotic arm in accordance with the present disclosure.

### Detailed description

Various exemplary embodiments and details are described below, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practised in any other embodiments even if not so illustrated, or if not so explicitly described.

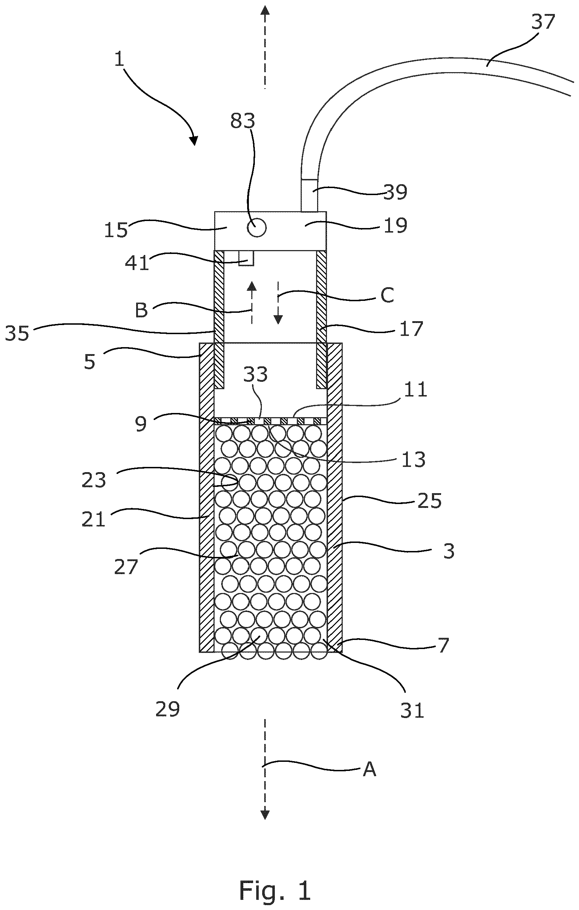

Fig. 1 shows a cross-sectional view of a dosing device 1 in accordance with the present disclosure, where the dosing device 1 comprises a dosing body 3 having a first end 5 and a second end 7 and having a longitudinal axis A. The dosing body 3 comprises a side wall 21 having an inner wall 23 and an outer wall 25. The dosing body 3 defines a dosing volume 27, where the dosing volume 27 is configured to receive a plurality of insects 29 inside the dosing volume 27 via a dosing opening 31.

The dosing device 1 comprises a filter part 9 having a first surface 11 and a second surface 13, where the first surface 11 faces the first end 5 of the dosing body 3, and the second surface 13 faces the second end 7 of the dosing body 3.

The dosing volume 27 is defined in a radial direction by the inner wall 23 of the dosing body 3, and in the longitudinal direction A towards the first end 5 is defined by the second surface 13 of the filter part 9. The dosing volume 27 is defined in the longitudinal direction A towards the second end 7 by the dosing opening 31, which provides access to the dosing volume 27 from the second end 7 of the dosing body 3.

The filter part 9 comprises at least one through-going opening 33, where the diameter of the through-going opening 33 is smaller than the smallest size of the insect 29 intended to be introduced into the dosing volume 27, ensuring that the insects 29 cannot pass in the longitudinal direction A through the through-going opening 33, and that the insects are held inside the dosing volume 27.

The dosing device 1 further comprises a valve device 15 which in this embodiment is attached to the first end 5 of the dosing body 3, where an outer surface 35 of an attachment part 17 may be attached to the inner wall 23 of the dosing body 3. The attachment part 17 may e.g. be a threaded coupling, where the inner wall 23 may comprise an inner thread, and the outer surface 35 of the attachment part 17 may comprise an outer thread which cooperates with the inner thread of the inner wall 23. The valve device 15 may comprise a valve part 19 which selectively can provide air pressure that is higher than the ambient air pressure, or can provide air pressure that is lower than the ambient air pressure. The valve part 17 may be connected to an air supply (not shown) via an air hose 37 which is connected to an air inlet 39. The air pressure may be provided with an air outlet 41 which is in fluid communication with the dosing volume 27 through the filter part 9, where the air pressure may be in the form of vacuum pressure to allow the insects 29 to be sucked into the dosing volume 27, or may be in the form of high positive pressure to allow the insects 29 to be dispensed out of the dosing volume 27.

The valve device may be provided with a valve part that is capable of converting positive air pressure to vacuum pressure, e.g. via a venturi nozzle, in one operational state, allowing air pressure to be utilized to suck up the insects 29 into the dosing volume 27 in the direction of an arrow B. The valve part 19 may further have a second operational state where the air pressure is directed towards the dosing volume 27 in the direction of an arrow C, where the insects 29 may be dispensed out of the dosing volume 27 following a change from the first operational state to the second operational state.

The valve device may be maintained in its first operational state, i.e. where the flow of air is in the direction of the arrow B, while the insects 29 are inside the dosing volume 27 in order to ensure that the insects 29 are maintained inside the dosing volume 27 while the dosing device may e.g. be moved from one position to another position.

The dosing device 1 may be utilized to collect a predefined number of insects 29 from a first predefined area to a second predefined area, e.g. in order to dose a predefined amount of insects 29 to a holding volume, or to dose a predefined amount of insects 29 to a predefined amount of feed.

The valve device may be provided with an electric or mechanical control interface 83 which may provide control signals to the valve device to control the operation of the valve device to collect and dispense the insects 29 in predefined manner. The control interface 83 may be connected to a switch or a control unit which provides the valve device with control input.

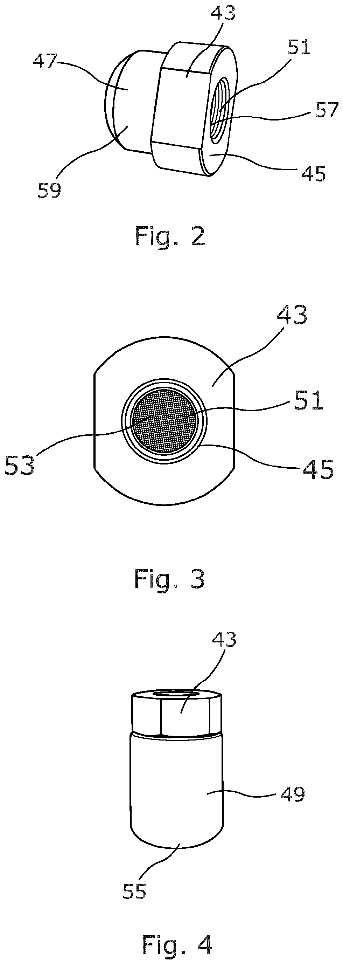

Figs. 2-4 show a filter part 43 having a first end 45 and a second end 47, where the filter part 43 may be detached from a dosing body 49, as shown in Fig. 4. The first end 45 may be provided with an opening 51 which extends from the first end 45 to the second end 47, where a filter device 53, such as a mesh, may be arranged in the opening 51 to provide fluid communication from the first end 45 to the second end 47 to allow air to flow from the first end 45 to the second end 47 and vice versa. The mesh may have openings that are sized to be smaller than the insects 29 that are intended to be held inside the dosing volume 27 (not shown) of the dosing body 49. The dosing body 49 may have a dosing opening 55 which allows the air to flow into and out of the dosing body 49 and having fluid communication with the opening 51 of the filter part 43. The first end 45 opening 51 of the filter part 43 may be provided with an inner thread 57 configured to allow the filter part 43 to be attached to an attachment part of a valve device. The second end 47 of the filter part 43 may have an outer surface 59 which is intended to be attached on an inner surface (not shown) of the dosing body 49. This type of filter part 43 allows the filter part 43 to be exchanged or removed from the valve device and/or the dosing body in order to clean or to be replaced by the second filter part 43.

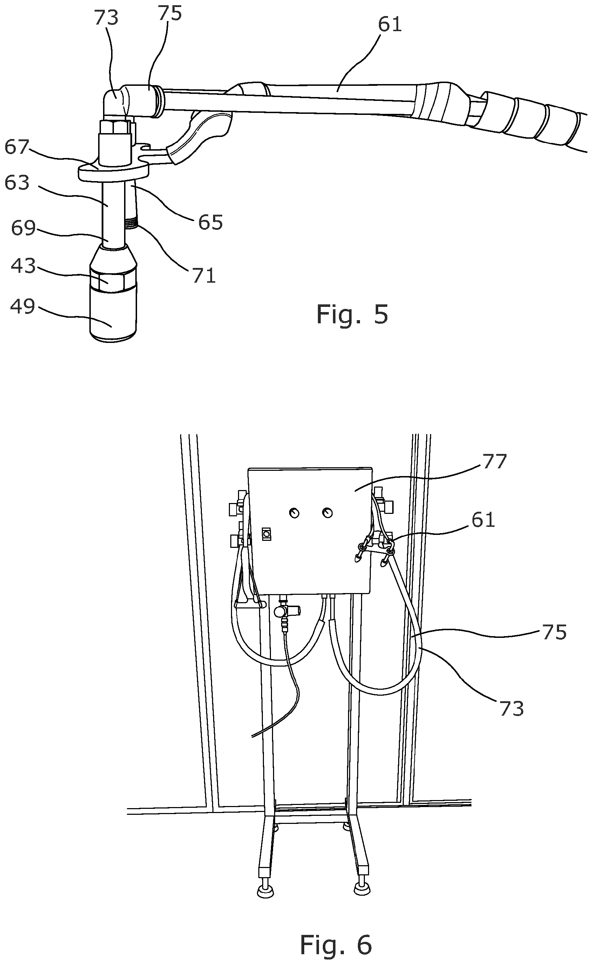

Fig. 5 shows a side view of an attachment part 61 which is part of the dosing device 27 and part of a valve device 77 as shown in Fig. 6. This attachment device 61 comprises a first attachment part 63 and a second attachment part 65 which are configured to be attached to the filter part 43 as shown in Figs. 2-4, where the filter part 43 is attached to a dosing body 49. The first attachment part 63 and the second attachment part 65 are fixed relative to each other via an attachment bracket 67. Free ends 69 of the attachment parts 63, 65 may be provided with an outer thread 71 which cooperates with the inner thread 57 of the filter part 43, as shown in Fig. 2. The opposing ends of the attachment parts 63, 65 may be connected to two separate air hoses 73, 75 which are in fluid communication with the valve device 77, allowing air to flow via the hoses 73, 75 and into the dosing bodies 49 to allow the insects 29 to be sucked into the dosing body 49 and into the dosing volume (not shown), and out of the dosing body 43 to dispense the insects 29 that have been sucked into the dosing body 49.

The valve device 77 shown in Fig. 6 is provided with a first pair of hoses 73, 75, as shown in Fig. 1, connected to the valve device 77, where the valve device 77 comprises a valve that is capable of providing vacuum force to the hoses 73, 75 in order to suck in the insects 29 into the dosing body 49, and where the valve device 77 is capable of providing air flow in the opposite direction to dispense the insects 29 from the dosing body 49. The valve device 77 may utilize an air hose 79 to provide air pressure into the valve device 77. The valve device 77 may utilize a VMECA MV10 or a VMECA M15 magic gripper device to provide vacuum and air pressure for the valve device 77, where the valve device 77 is modified to provide an air outlet to the dosing body 43. VMECA MV10 and MV15 are manufactured by VMECA Co., Ltd., 24 Beotkkot-ro 10-gil (Doksan-dong) Geumcheon-gu Seoul 08606, Korea (https://vmeca.com/en/product-category/magic-aripper-en/mv10-15-series-en/).

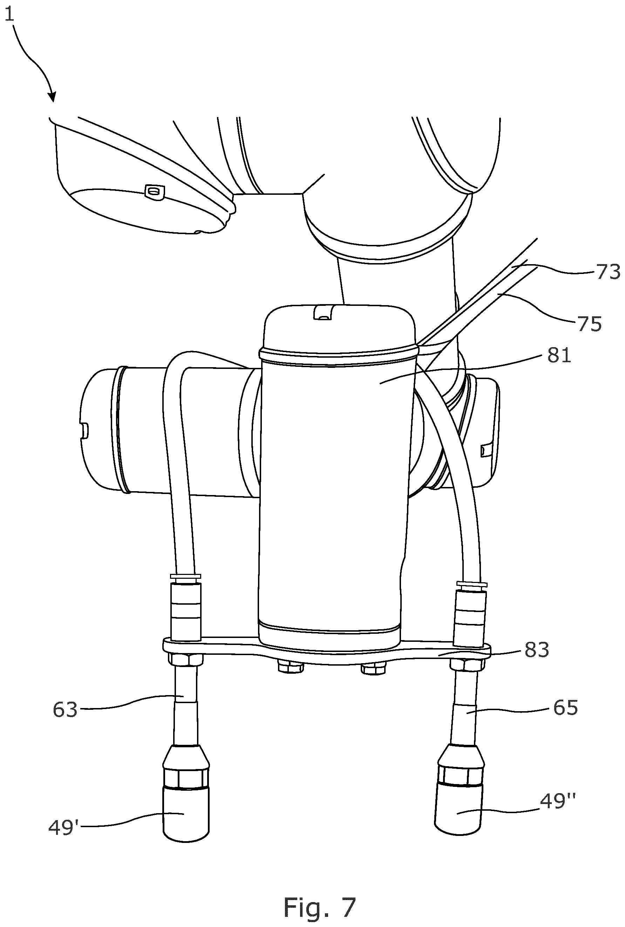

Fig. 7 shows a dosing device 1 attached to a robotic arm 81, where a first dosing body 49' and a second dosing body 49" are attached to a bracket 83 which is attached to an end of the robotic arm 81. The first dosing body 49' and the second dosing body 49" are connected to a first attachment part 63 and a second attachment part 65, and are connected to a valve device (not shown) via a first air hose 73 and a second air hose 75 respectively, which provides a suction force and a pushing force via air pressure that is controlled by the valve device 77.

The use of the terms "first", "second", "third" and "fourth", "primary", "secondary", "tertiary", etc., does not imply any particular order, but are included to identify individual elements. Moreover, the use of the terms "first", "second", "third" and "fourth", "primary", "secondary", "tertiary", etc., does not denote any order or importance, but rather the terms "first", "second", "third" and "fourth", "primary", "secondary", "tertiary", etc., are used to distinguish one element from another. Note that the words "first", "second", "third" and "fourth", "primary", "secondary", "tertiary", etc., are used here and elsewhere for labelling purposes only and are not intended to denote any specific spatial or temporal ordering.

Furthermore, the labelling of a first element does not imply the presence of a second element and vice versa.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs used do not limit the scope of the claims.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications and equivalents.

### List of references

- 1: Dosing device
- 3: Dosing body
- 5: First end
- 7: Second end
- 9: Filter part
- 11: First surface
- 13: Second surface
- 15: Valve device
- 17: Attachment part
- 19: Valve part
- 21: Side wall
- 23: Inner wall
- 25: Outer wall
- 27: Dosing volume
- 29: Insects
- 31: Dosing opening
- 33: Through-going opening
- 35: Outer surface
- 37: Air hose
- 39: Air inlet
- 41: Air outlet
- 43: Filter part
- 45: First end of filter part
- 47: Second end of filter part
- 49: Dosing body
- 51: Opening
- 53: Filter device
- 55: Dosing opening
- 57: Inner thread
- 59: Outer surface
- 61: Attachment part
- 63: First attachment part
- 65: Second attachment part
- 67: Attachment bracket
- 69: Free end of attachment part
- 71: Outer thread
- 73: Air hose
- 75: Air hose
- 77: Valve device
- 79: Air hose
- 81: Robotic arm
- 83: Bracket

## Claims

1. A dosing device for insects, the insects having a first size, and the dosing device comprising:
- a dosing body having a central axis having a first end facing the dosing device and a second end,
- a dosing volume having a longitudinal axis defined at least partly by the dosing body having a dosing opening arranged at the second end of the dosing body,
- a first valve device configured to control a passage of air into and/or out of the first end of the dosing body and/or into and/or out of the dosing volume, where the first valve device is configured to be in fluid communication with a first air source, and
- a filter part having a first surface facing the dosing opening and a second surface facing away from the dosing body, the first surface defining a boundary of the dosing volume, and the first end part having at least one through-going opening configured to allow air to pass, while preventing the passage of insects.

2. A dosing device in accordance with claim 1, wherein the at least one through-going opening has an opening diameter that is smaller than the first size of the insect.

3. A dosing device in accordance with claim 1 or 2, wherein the dosing body is a cylindrical body.

4. A dosing device in accordance with any of the preceding claims, wherein the filter part is arranged in fluid communication between the first valve device and/or the first dosing volume and/or the dosing body.

5. A dosing device in accordance with any of the preceding claims, wherein the filter part is removably attached to the dosing part and or the dosing device.

6. A dosing device in accordance with any of the preceding claims, wherein the filter part is in the form of a mesh-like structure having a plurality of through-going openings of substantially the same size.

7. A dosing device in accordance with any of the preceding claims, wherein the first valve device has an air supply inlet and a dosing device outlet and/or a dosing volume outlet.

8. A dosing device in accordance with any of the preceding claims, wherein the first valve device in a first operational state is configured to provide air pressure that is lower than the ambient air pressure.

9. A dosing device in accordance with any of the preceding claims, wherein the first valve device in a second operational state is configured to provide air pressure that is higher than the ambient air pressure.

10. A dosing device in accordance with any of the preceding claims, wherein, in a first operational state, the first valve device is configured to produce a vacuum which initiates a flow of air from the second end of the dosing body and/or the dosing opening of the dosing volume in a direction towards the filter part and/or the valve device. (through the dosing volume or the dosing body)

11. A dosing device in accordance with any of the preceding claims, wherein, in a second operational state, the first valve device is configured to produce a flow of air from the filter part and/or the valve device in a direction towards the second end of the dosing body and/or the dosing opening of the dosing volume. (through the dosing volume or the dosing body)

12. A dosing device in accordance with any of the preceding claims, wherein the dosing volume has a longitudinal length that is larger than the diameter of the dosing volume.

13. A dosing device in accordance with any of the preceding claims, wherein the dosing volume and/or the dosing body has a cylindrical shape.

14. A dosing device in accordance with any of the preceding claims, wherein the dosing volume and/or the dosing body has a first area having a first diameter and a second area having a second diameter, where the first diameter is different from the second diameter.

15. A dosing system comprising:
- a dosing device in accordance with claims 1-14, and
- a first air source in fluid communication with the first valve device.
